# EUROPEAN PATENT SPECIFICATION

(11) **EP 1 843 750 B1**
(45) Date of publication and mention of the grant of the patent: **16.10.2013**
(21) Application number: 06701649.3
(22) Date of filing: 30.01.2006
(51) Int. Cl.: A61K 9/12, A61K 31/37, A61K 31/46, A61P 11/06

(54) **NEBULIZER FORMULATION**
ZERSTÄUBERFORMULIERUNG
FORMULE POUR PULVÉRISATEUR

(30) Priority: 31.01.2005 GB 0501956
(43) Date of publication of application: 17.10.2007
(73) Proprietor: Breath Limited, Biggin Hill, Kent TN16 3TR (GB)
(72) Inventor: McAFFER, Ian, Gardner, Cameron, Breath Ltd, Biggin Hill,Kent TN16 3TR (GB); TASKO, Peter, Ernest, Resolution Chemicals Limited, Stevenage,Hertfordshire SG1 4QT (GB)
(74) Representative: Schlich, George
(86) International application number: PCT/GB2006/000309
(87) International publication number: WO 2006/079841

(56) References cited:
- WO-A-03/037159
- US-B1- 6 571 790
- BERGER W E: "LEVALBUTEROL: PHARMACOLOGIC PROPERTIES AND USE IN THE TREATMENT OF PEDIATRIC AND ADULT ASTHMA" ANNALS OF ALLERGY, ASTHMA & IMMUNOLOGY, ARLINGTON HEIGHTS, IL, US, vol. 90, no. 6, 1 June 2003 (2003-06-01), pages 583-591, XP009057491 ISSN: 1081-1206
- TRUITT T ET AL: "Levalbuterol compared to racemic albuterol: Efficacy and outcomes in patients hospitalized with COPD or asthma" CHEST, THE COLLEGE, CHICAGO, IL, US, vol. 123, no. 1, 2003, pages 128-135, XP002355128 ISSN: 0012-3692
- SLATTERY D ET AL: "LEVALBUTEROL HYDROCHLORIDE" PEDIATRIC PULMONOLOGY, JOHN WILEY, NEW YORK, NY, US, vol. 33, no. 2, 2002, pages 151-157, XP009057490 ISSN: 8755-6863

## Description

### Background to the Invention

The present invention relates to a nebulizer formulation, in particular a nebulizer formulation and a method of treatment for diseases such as Chronic Obstructive Pulmonary Disease (COPD) and asthma using the formulation.

Nebulizers provide a means of administering drugs to the airways of a patient whilst the patient breathes at an approximately normal rate. They are particularly suitable for patients who are unable, whether due to age or injury or otherwise, to inhale at the much higher rates required for administration of drugs via metered dose inhalers or dry powder inhalers and for patients who cannot for whatever reason coordinate the activation of the metered dose inhaler with their inhalation of breath. The nebulizer apparatus creates a vapour containing drug particles and the patient breathes the vapour via a mouthpiece or mask attached to the nebulizer. Typically, nebulizers are used to deliver drugs for the treatment of airways disorders such as asthma and COPD.

According to the U.S. Centers for Disease Control and Prevention, COPD is currently the fourth leading cause of death in the U.S. (behind heart disease, cancer and stroke), claiming the lives of in excess of 100,000 Americans annually. An estimated 16 million Americans have been diagnosed with some form of COPD, and as many as 16 million others have the condition but have not yet been diagnosed. COPD is hence regarded as a major and growing health care threat in the U.S. and throughout the rest of the world.

A known formulation for treatment of COPD comprises albuterol (also known as salbutamol) and ipratropium in an ampoule containing 3.0 ml of solution, and is described in WO 03/037159 and the equivalent US patent 6632842. In use, the contents of the ampoule are poured into the chamber of the nebulizer and the patient then breathes the vapour generated until the ampoule contents are used. Treatment is typically required up to 4 times per day, at regular intervals.

A known method for treating respiratory tract disorders and for increasing patient compliance comprising providing a nebulizer containing separate units of levalbuterol and ipratropium is described in US 6,571,790. The favourable therapeutic profile of levalbuterol compared to racemic albuterol is known from Berger 2003, Annals of Allergy Asthma and Immunology, 90:583-592, while a therapy combining levalbuterol and ipratropium is known from Truitt et al 2003, Chest, 123:128-135.

Low patient compliance is a known problem with nebulized drugs generally, as the period of nebulizing required to administer a dose is long, typically tens of minutes, perhaps half an-hour for a typical dose. Children and adults can become bored during this period. Patients who stop nebulizing prematurely do not receive a full dose. This can in turn lead to further reduced patient compliance as the inadequate dose fails to provide adequate therapy, discouraging further use.

It is an object of the present invention to provide formulations and their uses which overcome or at least ameliorate one or more of the above-identified problems.

### Summary of the Invention

The present invention provides novel nebulizer formulations, suitable for treatment of COPD, asthma and other conditions associated with reversible obstruction of the airways. The formulations can be utilized in a variety of known nebulizer apparatus with reduced wastage of ingredients and/or increased patient compliance.

The invention provides a method of treatment of COPD, asthma and other conditions associated with reversible obstruction of the airways comprising administering, via a nebulizer, a formulation containing both levalbuterol and ipratropium in a pharmaceutically acceptable carrier.

More specifically, the present invention provides a method of treatment of COPD, asthma and other conditions associated with reversible obstruction of the airways, comprising providing a nebulizer and an ampoule containing not more than 2.2 ml of a formulation comprising levalbuterol and ipratropium, in a pharmaceutically acceptable carrier, and administering the formulation using the nebulizer.

A filled ampoule of the invention contains a formulation of levalbuterol and ipratropium, and a more specific ampoule contains a nebulizer formulation, wherein the formulation contains from 1.0 to 1.5 mg of levalbuterol, and from 0.40 to 0.70 mg of ipratropium, in a total volume of from 0.3 to 2.2 ml.

Also provided by the invention is a method of increasing patient compliance in use of a nebulizer formulation, comprising providing a nebulizer, and an ampoule containing said formulation, wherein the formulation comprises levalbuterol and ipratropium in a pharmaceutically acceptable carrier and wherein the ampoule contains not more than 2.2 ml and not less than 0.3ml of said formulation, and administering the formulation using the nebulizer.

A kit of the invention comprises a formulation of the invention with instructions on how to use it.

### Detailed Description of the Preferred Embodiments

The invention enables use of nebulizer formulations without the lower limit on volume typically associated with the art. The beta agonist is formulated with and administered with ipratropium.

Reference to these active agents herein is intended to refer also to pharmaceutically acceptable derivatives thereof, such as but not limited to salts, esters, enol ethers, enol esters, acids, bases, solvates, hydrates or prodrugs thereof. Ipratropium is an anticholinergic agent and has a bronchodilator effect, and can thus aid delivery of, and act in synergy with the bronchodilator effect of, levalbuterol. Reference to ipratropium thus includes, but is not limited to, any form of ipratropium which has an anticholinergic effect in patients suffering from COPD, including, but not limited to, all automatic forms, enantiomer forms, stereoisomer, anhydrides, acid addition salts, base salts, solvates, analogues and derivatives of ipratropium.

Several acceptable salts of ipratropium exist and for the invention these may include, but are not limited to, halide salts such as bromide, chloride and iodide, described for example in U.S. Pat. No. 3,505,337, which is incorporated herein by reference.

A method of treatment of COPD or asthma using the teachings of the invention comprises administering to a human patient, via a nebulizer, a formulation containing effective amounts of both levalbuterol and ipratropium in a pharmaceutically acceptable carrier, and it is expected, using this ampoule formulation, to achieve improved acceptance of the medicine and better patient compliance.

A further anticipated advantage is that the concentration of the active ingredients can be increased, with the result that ampoules of the invention can contain reduced volume whilst retaining substantially the same unit dosage of drug to be administered. In one embodiment of the invention overall ampoule concentration (ie. including active and inactive ingredients) is substantially the same as in previous formulations but ampoule volume is approximately half. With reduced volume, there is reduced nebulizing time. This will further improve patient compliance as there is less time e.g. for patients to become bored or distracted whilst using the nebulizer.

In preferred embodiments of the invention, both ampoule volume is reduced, enabling reduced nebulising time, and ampoule concentration is reduced.

The compositions provided herein are used for treating, preventing, or ameliorating one or more symptoms of a bronchoconstrictive disorder or disease in a human subject. In one embodiment, the method includes nebulizer administration to a subject of an effective amount of a composition containing levalbuterol and ipratropium, whereby the disease or disorder is treated or prevented.

The invention relates in particular to formulations for treatment of COPD and asthma, including, but not limited to, chronic bronchitis, emphysema, and associated cor pulmonale (heart disease secondary to disease of the lungs and respiratory system) with pulmonary hypertension, right ventricular hypertrophy and right heart failure, and also bronchial asthma, allergic asthma and intrinsic asthma, e.g., late asthma and airway hyper-responsiveness.

The formulations of the present invention are designed for administration by nebulizer. A nebulized solution is one dispersed in air to form an aerosol, and a nebulizer generates very fine liquid droplets suitable for inhalation into the lung. Nebulizers typically use compressed air, ultrasonic waves, or a vibrating mesh to create a mist of the droplets and may also have a baffle to remove larger droplets from the mist by impaction. A variety of nebulizers are available for this purpose, such as ultrasonic nebulizers, jet nebulizers and breath-actuated nebulizers. In use, mouthpieces or masks are typically attached to a patient to aid delivery of the nebulized solution.

In preferred embodiments of the invention, formulations are for delivery with and patients are treated using a high efficiency nebulizer, in particular one that can deliver at least 15%, preferably at least 25%, more preferably at least 35% of the drug substance to the patient's lungs.

In specific embodiments of the invention, formulations are delivered using a high efficiency jet nebulizer, a high efficiency ultrasonic nebulizer or a high efficiency vibrating mesh nebulizer, use of these devices enabling and / or enhancing the use of the reduced volume formulations of the invention. Jet nebulizers are particularly preferred, and one example is the PARI LC Plus (registered trade mark, Pari GmbH, Germany) nebulizer.

The invention is of particular application to COPD. Specifically, a method of treatment of COPD according to the present invention hence comprises:-
(1) providing:-
   a) a nebulizer, and
   b) an ampoule containing not more than 2.2 ml of a formulation comprising levalbuterol and ipratropium in a pharmaceutically acceptable carrier, and
(2) administering the formulation using the nebulizer.

Preferably, the invention provides and uses ampoules which contain not less than 0.5 ml of formulation, more preferably 1.0 to 2 ml of said formulation, and very preferably 1.5ml to 2 ml of said formulation. These reduced volumes can lead to significant reductions in treatment times, with the expected advantages explained.

Formulations and compositions of the invention generally comprise a pharmaceutically acceptable carrier. The carrier is preferably a liquid carrier. Further, the carrier preferably comprises water and may comprise other components.

A filled ampoule of the invention contains a formulation of levalbuterol and ipratropium. This is generally in a pharmaceutically acceptable carrier and buffered for human use to a pH of 3.5 - 5.5. The formulations of the examples are buffered to about pH 4. The levalbuterol is used in the examples as the HCl salt, though other salts are suitable, and the ipratropium is generally used as its HBr salt, though again other salts are suitable. HCl is conveniently used for pH adjustment. The formulations are free of preservative, which is an advantage as some preservatives can be associated with bronchoconstrictor effects - the opposite effect to that required by the formulation. Water is used to provide the carrier, and water for injection is preferred due to its purity.

One or more tonicity adjusting agents may be added to provide the desired ionic strength. Tonicity adjusting agents for use herein include those which display no or only negligible pharmacological activity after administration. Both inorganic and organic tonicity adjusting agents may be used. Compositions of the invention can also include excipients and/or additives. Examples of these are surfactants, stabilizers, complexing agents, antioxidants, or preservatives which prolong the duration of use of the finished pharmaceutical formulation, flavorings, vitamins, or other additives known in the art. Complexing agents include, but are not limited to, ethylenediaminetetraacetic acid (EDTA) or a salt thereof, such as the disodium salt, citric acid, nitrilotriacetic acid and the salts thereof. In one embodiment, the complexing agent is EDTA. Preservatives include, but are not limited to, those that protect the solution from contamination with pathogenic particles, including benzalkonium chloride or benzoic acid, or benzoates such as sodium benzoate. Antioxidants include, but are not limited to, vitamins, provitamins, ascorbic acid, vitamin E or salts or esters thereof.

Formulations as described in this invention can be readily prepared by a person of skill in the art. In one method, a solution of NaCl is prepared with concentration approximately 9g/l. To this are added ipratropium bromide to a concentration as desired, but typically about 0.25mg/ml and levalbuterol, again to the concentration desired but typically about 0.625 mg/ml. HCl is then added to give a final pH of about 4.0. This formulation can be filled into ampoules using blow fill seal technology (described in more detail below) to yield ampoules with the required extractable volume of formulation.

Reference to an ampoule with a specified volume and to the extractable volume of an ampoule refer to the volume of solution that can be extracted from the ampoule in normal use, e.g. by breaking it open and pouring out the contents without actively flushing the ampoule or carrying out scientific extraction methods. There is in addition some tolerance in the volumes recited, as filling machines vary in their accuracy. By way of illustration, "an ampoule containing 3ml of solution" and a "3ml ampoule", say, both refer to an ampoule which contains about 3.1 to about 3.2, generally about 3.15, ml of solution and which when opened and poured into the nebulizer results in approximately 3ml of solution being transferred into the nebulizer. Hence, the volumes recited refer to the amount of solution that can be readily extracted from the ampoule rather than the amount the ampoule is filled with.

Preferred embodiments of the invention provide methods and ampoules for adults, for whom a typical dose of levalbuterol is about 1.25 milligrams. A typical dose of ipratropium is about 0.5 milligrams. Ampoules thus suitably contain such amounts of the active ingredients in their extractable volume.

Ampoules of the invention have reduced volume, containing 2.2ml or less of said formulation, preferably 2.0ml or less of said formulation or 1.0 to 2 ml of said formulation. Specific embodiments of the invention, set out in detail below, provide ampoules of about 2ml. Other suitable ampoule volumes are about 1.5ml, about 1.0ml and about 0.5ml.

In use of the formulations of the invention, it is possible to deliver a sufficient dose of ipratropium and levalbuterol in a shorter period of time than is necessary for known combination formulations. Hence, the invention provides in another aspect a method of increasing patient compliance in use of a nebulizer formulation, comprising
(1) providing:-
   a) a nebulizer, and
   b) an ampoule containing said formulation, wherein the formulation comprises levalbuterol and ipratropium in a pharmaceutically acceptable carrier and wherein the ampoule contains not more than 2.2 ml and not less than 0.3ml of said formulation,
      and
(2) administering the formulation using the nebulizer.

The volume of the ampoule can be reduced following the teachings of the invention. However, there is a practical limit to the concentration of the contents of ampoules of the invention in as much as very small amounts of highly concentrated liquids are easily spilled and are not so easy to dispense accurately. In preferred methods and formulations, the ampoules contain not more than 2ml and not less than 0.5 ml of said formulation.

The formulation used in the method typically contains from 0.75 to 2.0 mg of levalbuterol, preferably from 1.0 to 1.5 mg of levalbuterol. The formulation also typically contains from 0.25 to 1.0 mg of ipratropium, preferably from 0.40 to 0.70 mg of ipratropium. These formulations preferably have volumes of about 2.0ml, about 1.5ml, about 1.0ml or about 0.5ml.

A specific ampoule contains a nebulizer formulation of:-
a) from 1.0 to 1.5 mg of levalbuterol, and
b) from 0.40 to 0.70 mg of ipratropium,
in a volume of from 0.3 to 2.2 ml.

The total volume is preferably from 0.5ml to 2 ml, and more preferably has a total volume of about 2 ml or about 1.5ml.

In use of the invention, increased concentration of the active ingredients, ipratropium and levalbuterol, enables smaller volumes of the formulation to be used. Generally, the levalbuterol is present at about 0.57 mg/ml or higher, about 0.63 mg/ml or higher or about 0.83 mg/ml or higher. An ampoule with an extractable volume of 0.5ml can contain levalbuterol at about 2.5mg/ml. The ipratropium is generally present at a concentration of about 0.23 mg/ml or higher, about 0.25 mg/ml or higher or about 0.33 mg/ml or higher. An ampoule with an extractable volume of 0.5ml can contain ipratropium at about 1 mg/ml.

Pharmaceutical compositions containing levalbuterol and an anticholinergic agent for administration via nebulization are hence provided. The compositions are preferably sterile filtered and filled in ampoules or vials, including unit dose vials, providing sterile unit dose formulations for use in a nebulizer.

In a further embodiment, the present invention provides a container containing a vial, comprising a single unit dose of a therapeutically effective amount of levalbuterol and ipratropium in a sterile solution, or a plurality of such vials.

The extractable volume of each unit dose of a specific embodiment of the invention comprises about 1.25mg of levalbuterol (or equivalent amount of a derivative thereof) and about 0.5 mg ipratropium bromide (or equivalent amount of a derivative thereof) in a sterile, aqueous solution. The solution contains sodium chloride at about 9mg/ml to make the solution isotonic and hydrochloric acid to adjust pH of the solution to about 4.0. It is optional to include a chelating agent, such as EDTA. The volume is preferably about 2.0ml or about 1.5ml.

The invention additionally provides kits for use in treatment of the diseases described herein. The kits comprise:-
(1) a container, containing a single unit dose of a therapeutically effective amount of levalbuterol plus ipratropium; and
(2) instructions on how the dose is to be used.

The single unit dose is suitably as described elsewhere herein in relation to formulations of the invention. The instructions instruct the patient as to how the dose should be used in conjunction with a nebulizer, such as how to open it and transfer its contents into the nebulizer, how to operate the nebulizer and for how long nebulizing should be continued to complete administration of the dose.

Kits of the invention can contain a plurality of single unit doses. One kit comprises at least 120 or at least 125 single unit doses, being designed to provide one month's worth of doses to be taken 4 times per day. Another kit comprises at least 25 or at least 28 single unit doses, designed for a week's supply at 4 per day. Other kits may usefully contain 30 or 60 single unit doses.

For embodiments of the invention in which the extractable volume is 2.2ml or less, especially where the volume is 2.0ml or 1.5ml or lower, the instructions may explain that the present formulation can be administered in less time than a previously known formulation, such as a known 3ml formulation, hence reinforcing this advantage of the invention and improving the prospects for increased patient compliance. Preferably, the instructions explain that the patient should continue administering the dose until the complete dose has been administered.

Formulations of the invention are suitable for filing into ampoules using "blow fill seal" (BFS) methods. The principle is that a plastic parison is extruded from polymer, formed into a container, filled and sealed in a single aseptic operation. BFS is now the preferred method for aseptic manufacture of ampoules due to the flexibility in container design, overall product quality, product output and low operational costs. Fill accuracies of better than ±5% have been demonstrated for container volumes as small as 0.5ml and hence BFS is suitable for manufacture of ampoules according to the invention.

One BFS operation includes the multi-step process of blow moulding, aseptic filling and hermetic sealing of liquid products with fill volumes ranging from 0.1ml to 1,000ml, though for ampoules volumes in the range 0.5ml to 5ml are more common. A variety of polymers may be used in the process; low and high-density polyethylene and polypropylene are the most popular.

Furthermore, the BFS process flow is normally impacted by only two raw materials - product and polymer - that are each processed inline, thereby making the process amenable to large uninterrupted batch sizes, some in excess of 500,000 or 1,000,000 units, and fill durations of up to 120 hours.

In a typical operation, to form the container, thermoplastic is continuously extruded in a tubular shape. When the tube reaches the proper length, the mould closes and the parison is cut. The bottom of the parison is pinched, closed and the top is held in place with a set of holding jaws. The mould is then transferred to a position under the filling station. To fill the container, the nozzle assembly lowers into the parison until the nozzles form a seal with the neck of the mould. Container formation is completed by applying vacuum on the mould side of the container and by blowing sterile filtered air into the interior of the container. The fill system delivers a precise dosage of product into the container. The nozzles retract into their original position. Lastly, to seal the container, following completion of the filling process, the top of the container remains semi-molten. Separate seal moulds close to form the top and hermetically seal the container. The moulds open and the container is then conveyed out of the machine. The whole of the above process is operated in pharmaceutical aseptic processing conditions.

BFS machines are commercially available from a number of suppliers, including Weiler Engineering, Inc (US) and rommelag USA Inc (US).

The invention is now illustrated in the following non-limiting examples.

### Examples

### Example 1

A bulk nebulizer formulation was prepared having the following composition:-

| Component | Amount / Concentration |
|---|---|
| Levalbuterol | 0.625 mg/ml |
| Ipratropium | 0.25 mg/ml |
| NaCl solution (for injection) | 9mg/ml |
| HCl | To adjust pH to 4.0 |

The formulation was loaded into 2 ml (extractable volume), twist-top plastic ampoules.

### Example 2

A bulk nebulizer formulation was prepared having the following composition:-

| Component | Amount / Concentration |
|---|---|
| Levalbuterol | 0.83 mg/ml |
| Ipratropium | 0.33 mg/ml |
| NaCl solution (for injection) | 9mg/ml |
| HCl | To adjust pH to 4.0 |

The formulation was loaded into 1.5 ml (extractable volume), twist-top plastic ampoules.

The invention hence provides nebulizer formulations and uses thereof.

## Claims

1. Use of a formulation comprising levalbuterol and ipratropium in a pharmaceutically acceptable carrier in the manufacture of a medicament for the treatment of COPD or asthma wherein the medicament is administered via a nebuliser ampoule containing not more than 2.2ml of said formulation.

2. The use of claim 1, wherein the ampoule contains not less than 0.5 ml of said formulation.

3. The use of claim 1, wherein the ampoule contains 1.0 to 2 ml of said formulation.

4. The use of claim 1, wherein the ampoule contains 1.5ml to 2 ml of said formulation.

5. The use of claim 1, wherein the medicament is for treatment of asthma.

6. The use of claim 1, wherein the medicament is for treatment of COPD.

7. The use of any of claims 1-6, wherein the formulation contains about 1.25 milligrams of levalbuterol.

8. The use of any of claims 1-7, wherein the formulation contains about 0.5 milligrams of ipratropium.

9. A filled ampoule containing a formulation comprising levalbuterol and ipratropium, and a pharmaceutically acceptable carrier, wherein the ampoule contains not more than 2.2ml of said formulation.

10. The filled ampoule of claim 9, containing 2.0ml or less of said formulation.

11. The filled ampoule of claim 9, containing 1.0 to 2 ml of said formulation.

12. Use of a formulation comprising levalbuterol and ipratropium in a pharmaceutically acceptable carrier in the manufacture of a medicament for increasing patient compliance in use of a nebuliser formulation wherein the medicament is administered via an ampoule containing not more than 2.2 ml and not less than 0.3ml of said formulation.

13. The use of claim 12, wherein the ampoule contains not more than 2ml and not less than 0.5 ml of said formulation.

14. The use of claim 12, wherein the formulation contains from 0.75 to 2.0 mg of levalbuterol.

15. The use of claim 12, wherein the formulation contains from 1.0 to 1.5 mg of levalbuterol.

16. The use of any of claims 12-15, wherein the formulation contains from 0.25 to 1.0 mg of ipratropium.

17. The use of any of claims 12-15, wherein the formulation contains from 0.4 to 0.7 mg of ipratropium.

18. A kit comprising:-
(1) an ampoule, containing a single unit dose of 2.2ml or less in volume of a therapeutically effective amount of levalbuterol and ipratropium, and a pharmaceutically acceptable carrier; and
(2) instructions on how the dose is to be used.

19. The kit of claim 18, wherein and the instructions explain that the single unit dose can be administered in less time than a previously known formulation.

20. The kit of claim 18 or 19, wherein the instructions explain that the patient should continue administering the dose until the complete dose has been administered.

21. The kit of any of claims 18-20, wherein the single unit dose is about 2.0ml in volume.

22. The kit of claim 21, wherein the volume is about 1.5ml

23. The kit of any of claims 18-22, comprising instructions to deliver the dose using a high efficiency nebuliser.

24. The kit of claim 21 wherein the nebuliser is a jet nebuliser.

25. The kit of any of claims 18-24, comprising at least 25 of said single unit doses.

26. The kit of claim 25, comprising at least 120 of said single unit doses.

27. A formulation comprising levalbuterol and ipratropium in a pharmaceutically acceptable carrier for use in the treatment of COPD or asthma wherein the medicament is administered via a nebuliser ampoule containing not more than 2.2ml of said formulation.

## Patentansprüche

1. Verwendung einer Formulierung, die Levalbuterol und Ipratropium in einem pharmazeutisch annehmbaren Träger umfasst, in der Herstellung eines Medikaments für die Behandlung von COPD oder Asthma, wobei das Medikament über eine Verneblerampulle, die nicht mehr als 2,2 ml der Formulierung enthält, verabreicht wird.

2. Verwendung nach Anspruch 1, wobei die Ampulle nicht weniger als 0,5 ml der Formulierung enthält.

3. Verwendung nach Anspruch 1, wobei die Ampulle 1,0 bis 2 ml der Formulierung enthält.

4. Verwendung nach Anspruch 1, wobei die Ampulle 1,5 ml bis 2 ml der Formulierung enthält.

5. Verwendung nach Anspruch 1, wobei das Medikament für die Behandlung von Asthma ist.

6. Verwendung nach Anspruch 1, wobei das Medikament für die Behandlung von COPD ist.

7. Verwendung nach einem der Ansprüche 1 bis 6, wobei die Formulierung etwa 1,25 Milligramm Levalbuterol enthält.

8. Verwendung nach einem der Ansprüche 1 bis 7, wobei die Formulierung etwa 0,5 Milligramm Ipratropium enthält.

9. Gefüllte Ampulle, die eine Formulierung enthält, die Levalbuterol und Ipratropium und einen pharmazeutisch annehmbaren Träger umfasst, wobei die Ampulle nicht mehr als 2,2 ml der Formulierung enthält.

10. Gefüllte Ampulle nach Anspruch 9, die 2,0 ml oder weniger der Formulierung enthält.

11. Gefüllte Ampulle nach Anspruch 9, die 1,0 bis 2 ml der Formulierung enthält.

12. Verwendung einer Formulierung, die Levalbuterol und Ipratropium in einem pharmazeutisch annehmbaren Träger umfasst, in der Herstellung eines Medikaments zur Erhöhung der Patientenverträglichkeit im Einsatz einer Verneblerformulierung, wobei das Medikament über eine Ampulle, die nicht mehr als 2,2 ml und nicht weniger als 0,3 ml der Formulierung enthält, verabreicht wird.

13. Verwendung nach Anspruch 12, wobei die Ampulle nicht mehr als 2 ml und nicht weniger als 0,5 ml der Formulierung enthält.

14. Verwendung nach Anspruch 12, wobei die Formulierung von 0,75 bis 2,0 mg Levalbuterol enthält.

15. Verwendung nach Anspruch 12, wobei die Formulierung von 1,0 bis 1,5 mg Levalbuterol enthält.

16. Verwendung nach einem der Ansprüche 12 bis 15, wobei die Formulierung von 0,25 bis 1,0 mg Ipratropium enthält.

17. Verwendung nach einem der Ansprüche 12 bis 15, wobei die Formulierung von 0,4 bis 0,7 mg Ipratropium enthält.

18. Ausrüstungssatz (Kit), der umfasst:
(1) eine Ampulle, die eine einzelne Einheitsdosis von 2,2 ml oder weniger im Volumen einer therapeutisch wirksamen Menge von Levalbuterol und Ipratropium und einen pharmazeutisch annehmbaren Träger enthält; und
(2) Anweisungen, wie die Dosis zu verwenden ist.

19. Ausrüstungssatz nach Anspruch 18, wobei die Anweisungen erklären, dass die einzelne Einheitsdosis in einer kürzeren Zeitspanne als eine ehemals bekannte Formulierung verabreicht werden kann.

20. Ausrüstungssatz nach Anspruch 18 oder 19, wobei die Anweisungen erklären, dass der Patient so lange mit der Verabreichung der Dosis fortfahren sollte, bis die vollständige Dosis verabreicht worden ist.

21. Ausrüstungssatz nach einem der Ansprüche 18 bis 20, wobei die einzelne Einheitsdosis etwa 2,0 ml im Volumen beträgt.

22. Ausrüstungssatz nach Anspruch 21, wobei das Volumen etwa 1,5 ml beträgt.

23. Ausrüstungssatz nach einem der Ansprüche 18 bis 22, der Anweisungen umfasst, unter Verwendung eines Hochleistungsverneblers die Dosis abzugeben.

24. Ausrüstungssatz nach Anspruch 21, wobei der Vernebler ein Strahlvernebler ist.

25. Ausrüstungssatz nach einem der Ansprüche 18 bis 24, der mindestens 25 der einzelnen Einheitsdosen umfasst.

26. Ausrüstungssatz nach Anspruch 25, der mindestens 120 der einzelnen Einheitsdosen umfasst.

27. Formulierung, die Levalbuterol und Ipratropium in einem pharmazeutisch annehmbaren Träger für die Verwendung in der Behandlung von COPD oder Asthma umfasst, wobei das Medikament über eine Verneblerampulle, die nicht mehr als 2,2 ml der Formulierung enthält, verabreicht wird.

## Revendications

1. Utilisation d'une formulation comprenant du levalbutérol et de l'ipratropium dans un support pharmaceutiquement acceptable dans la fabrication d'un médicament pour le traitement d'une MPOC ou de l'asthme, dans laquelle le médicament est administré par l'intermédiaire d'une ampoule pour pulvérisateur ne contenant pas plus de 2,2 ml de ladite formulation.

2. Utilisation selon la revendication 1, dans laquelle l'ampoule ne contient pas moins de 0,5 ml de ladite formulation.

3. Utilisation selon la revendication 1, dans laquelle l'ampoule contient 1,0 à 2 ml de ladite formulation.

4. Utilisation selon la revendication 1, dans laquelle l'ampoule contient 1,5 ml à 2 ml de ladite formulation.

5. Utilisation selon la revendication 1, dans laquelle le médicament est pour le traitement de l'asthme.

6. Utilisation selon la revendication 1, dans laquelle le médicament est pour le traitement d'une MPOC.

7. Utilisation selon l'une quelconque des revendications 1 à 6, dans laquelle la formulation contient environ 1,25 milligramme de levalbutérol.

8. Utilisation selon l'une quelconque des revendications 1 à 7, dans laquelle la formulation contient environ 0,5 milligramme d'ipratropium.

9. Ampoule remplie contenant une formulation comprenant du levalbutérol et l'ipratropium, et un support pharmaceutiquement acceptable, dans laquelle l'ampoule ne contient pas plus de 2,2 ml de ladite formulation.

10. Ampoule remplie selon la revendication 9, contenant 2,0 ml ou moins de ladite formulation.

11. Ampoule remplie selon la revendication 9, contenant 1,0 à 2 ml de ladite formulation.

12. Utilisation d'une formulation comprenant du levalbutérol et de l'ipratropium dans un support pharmaceutiquement acceptable dans la fabrication d'un médicament pour augmenter l'assiduité du patient à utiliser une formulation en pulvérisateur dans laquelle le médicament est administré par l'intermédiaire d'une ampoule ne contenant pas plus de 2,2 ml et pas moins de 0,3 ml de ladite formulation.

13. Utilisation selon la revendication 12, dans laquelle l'ampoule ne contient pas plus de 2 ml et pas moins de 0,5 ml de ladite formulation.

14. Utilisation selon la revendication 12, dans laquelle la formulation contient de 0,75 à 2,0 mg de levalbutérol.

15. Utilisation selon la revendication 12, dans laquelle la formulation contient de 1,0 à 1,5 mg de levalbutérol.

16. Utilisation selon l'une quelconque des revendications 12 à 15, dans laquelle la formulation contient de 0,25 à 1,0 mg d'ipratropium.

17. Utilisation selon l'une quelconque des revendications 12 à 15, dans laquelle la formulation contient de 0,4 à 0,7 mg d'ipratropium.

18. Kit comprenant :
(1) une ampoule, contenant une dose unitaire unique de 2,2 ml ou moins en volume d'une quantité thérapeutiquement efficace de levalbutérol et d'ipratropium, et un support pharmaceutiquement acceptable ; et
(2) des instructions sur la façon d'utiliser la dose.

19. Kit selon la revendication 18, dans lequel et les instructions expliquent que la dose unitaire unique peut être administrée en moins de temps qu'une formulation connue précédemment.

20. Kit selon la revendication 18 ou la revendication 19, dans lequel les instructions expliquent que le patient doit continuer à administrer la dose jusqu'à ce que la dose complète soit administrée.

21. Kit selon l'une quelconque des revendications 18 à 20, dans lequel la dose unitaire unique est d'environ 2,0 ml en volume.

22. Kit selon la revendication 21, dans lequel le volume est d'environ 1,5 ml.

23. Kit selon l'une quelconque des revendications 18 à 22, comprenant des instructions pour administrer la dose en utilisant un vaporisateur hautement efficace.

24. Kit selon la revendication 21, dans lequel le vaporisateur est un vaporisateur à jet.

25. Kit selon l'une quelconque des revendications 18 à 24, comprenant au moins 25 desdites doses unitaires uniques.

26. Kit selon la revendication 25, comprenant au moins 120 desdites doses unitaires uniques.

27. Formulation comprenant du levalbutérol et de l'ipratropium dans un support pharmaceutiquement acceptable à utiliser dans le traitement d'une MPOC ou de l'asthme, dans laquelle le médicament est administré par l'intermédiaire d'une ampoule pour pulvérisateur ne contenant pas plus de 2,2 ml de ladite formulation.
